Europäisches Patentamt

European Patent Office

Office européen des brevets

(11) Veröffentlichungsnummer: **0 511 583 A2**

## (12) EUROPÄISCHE PATENTANMELDUNG

(21) Anmeldenummer: **92106897.9**

(51) Int. Cl.5: **G01N 33/00**, G01N 1/24

(22) Anmeldetag: **22.04.92**

(30) Priorität: **30.04.91 DE 9105354 U**

(43) Veröffentlichungstag der Anmeldung:
**04.11.92 Patentblatt 92/45**

(84) Benannte Vertragsstaaten:
**AT DE FR GB IT SE**

(71) Anmelder: **SIEMENS AKTIENGESELLSCHAFT**
**Wittelsbacherplatz 2**
**W-8000 München 2(DE)**

(72) Erfinder: **Krämer, Rolf**
**Neureuter Hauptstrasse 6**
**W-7500 Karlsruhe 21(DE)**

(54) **Einrichtung zur Aufbereitung von mit Russpartikeln beladenen Abgasen für die Analyse.**

(57) In den Abgasstrom ist ein Pyrolysefilter eingeschaltet, bestehend aus einer elektrisch aufheizbaren
Filterkerze (2) aus metallischem Geflecht oder Gewebe. In vorgegebenen Zeitabständen wird die Abgaszufuhr gesperrt und unter Zufuhr eines Inertgases (N2) das Filter (2) aufgeheizt, um die an den
Rußpartikeln angelagerten Kohlenwasserstoffe freizusetzen. In einer weiteren Betriebsphase wird das
Filter (2) weiter aufgeheizt, so daß die auf ihm befindlichen Rußpartikel unter Zufuhr von Luft verbrannt werden, um den Rußgehalt des Abgases mittels einer $CO_2$-Messung festzustellen.

Die Erfindung wird bei der Abgasanalyse von
Motoren, insbesondere Dieselmotoren, eingesetzt.

EP 0 511 583 A2

Die Erfindung betrifft eine Einrichtung zur Aufbereitung von mit Rußpartikeln beladenen Abgasen für die Analyse.

Derartige Abgase, insbesondere Abgase von Ölfeuerungen und Dieselmotoren, müssen aus Gründen des Umweltschutzes überwacht werden, um eine optimale Einstellung der Verbrennung und damit eine Verminderung der Schadstoffbelastung zu erreichen. Bevor mit gasanalytischen Methoden die Anteile an Kohlenwasserstoffen, Kohlenoxyd, Kohlendioxyd, Stickoxyden und Sauerstoff im Abgas ermittelt werden, sind die mitgeführten Rußpartikel zu entfernen. Dies geschieht in bekannter Weise durch Rußfilter.

Es hat sich jedoch herausgestellt, daß sich unverbrannte Kohlenwasserstoffe im Abgas an die Rußpartikel anlagern, so daß diese Kohlenwasserstoffe bei der folgenden Messung nicht erfaßt werden.
Da die Umweltbelastung durch die Rußpartikel mit zunehmender Kohlenwasserstoffbelastung steigt, ist die Möglichkeit zu schaffen, auch diese Kohlenwasserstoffe meßtechnisch zu erfassen.

Die Erfindung, wie sie in den Ansprüchen gekennzeichnet ist, löst somit die Aufgabe, eine Einrichtung zur Aufbereitung von mit Rußpartikeln beladenen Abgasen für die Analyse zu schaffen, die möglichst einfach aufgebaut ist und welche die an die Rußpartikel gebundenen Kohlenwasserstoffe freisetzt. In einer weiteren Betriebsphase können die auf dem Filter angesammelten Rußpartikel unter Zufuhr von Luft pyrolysiert und über eine $CO_2$-Messung mengenmäßig bestimmt werden.

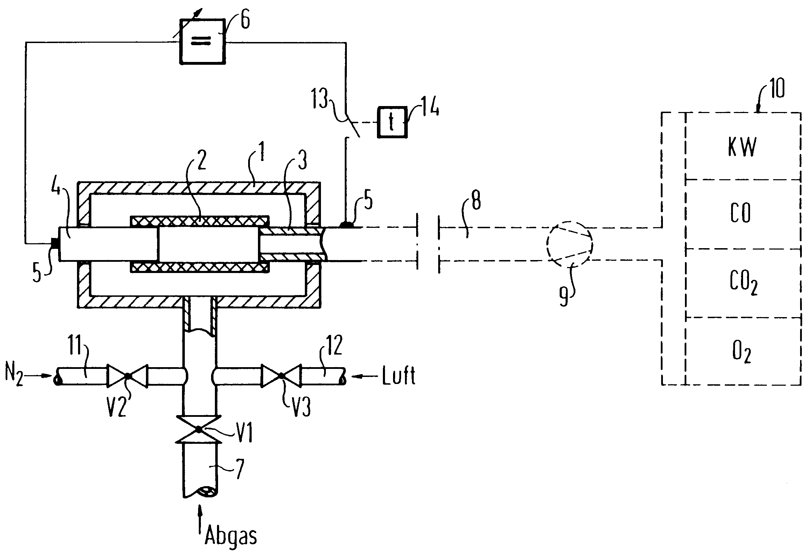

Zur Erläuterung der Erfindung ist in der Figur ein Ausführungsbeispiel dargestellt und im folgenden beschrieben.

In einer zylindrischen Filterkammer 1 aus elektrisch isolierendem Werkstoff ist koaxial ein aus elektrisch leitendem, korrosionsfestem und temperaturbeständigem Werkstoff, beispielsweise Edelstahl, bestehendes, hohlzylindrisches Filterelement angeordnet, welches an einem Ende auf ein durch die eine Stirnseite der Filterkammer 1 geführtes metallisches Rohr 3 aufgesteckt und befestigt ist, während sein anderes Ende auf einen Metallstab 4 aufgesteckt und so nach Art einer Filterkerze verschlossen ist. Der Metallstab 4 ist durch die andere Stirnseite der Filterkammer 1 nach außen geführt.
Das Filterelement 2 besteht aus einem Metallgewebe oder -vlies mit einer Porenweite von etwa 5 $\mu$m. An den äußeren Teilen von Rohr 3 und Metallstab 4 sind Anschlüsse 5 zur Zuführung von elektrischer Energie aus einer einstellbaren Energiequelle 6 angebracht, so daß das Filterelement 2 als aufheizbarer, ohmscher Widerstand betrieben werden kann. Mit einem Schalter 13, der von einem Zeitschaltwerk 14 betätigt wird, läßt sich die Energiezufuhr steuern.

Das zu untersuchende Abgas wird über eine mittels eines Ventils V1 absperrbare Zuführung 7 in die Filterkammer 1 eingeführt. Dort lagern sich die mitgeführten Rußpartikel auf der Oberfläche des Filterelements 2 an. Das von Partikeln gereinigte Abgas tritt in das Innere des Filterelements 2 und strömt von dort über das Rohr 3 und eine angeschlossene Rohrleitung 8 mit Saugpumpe 9 einem Analysensystem 10 zu. Wie angedeutet, enthält dieses Analysensystem 10 Aggregate zur Bestimmung von Kohlenwasserstoffen (KW), z. B. einen Flammenionisationsdetektor, sowie nichtdispersive Infrarot-Gasanalysatoren zur Bestimmung von Kohlenoxyd (CO) und Kohlendioxyd ($CO_2$) und einen elektrochemischen Meßfühler für die Bestimmung von Sauerstoff ($O_2$).

In die Abgaszuführung 7 münden zwischen Ventil V1 und der Filterkammer 1 zwei weitere Zuführungen, nämlich eine mittels des Ventils V2 absperrbare Zuführung 11 für Stickstoff ($N_2$) und eine mittels eines Ventils V3 absperrbare Zuführung 12 für Luft.

Ein Meßzyklus umfaßt drei Betriebsphasen.
Betriebsphase 1: Das Abgas strömt über die Zuführung 7 bei geöffnetem Ventil V1 in die Filterkammer 1, die Rußpartikel setzen sich auf dem Filterelement 2 ab und das Gas strömt über das Rohr 3 und die Rohrleitung 8 in das Analysensystem 10. Die Ventile V2 und V3 in den Zuleitungen 11 und 12 sind geschlossen.

Betriebsphase 2: Nach einer vorgegebenen Zeit schließt das programmierbare Zeitschaltwerk 14 den bisher offenen Schalter 13, so daß elektrische Energie über die Anschlüsse 5 dem Filterelement 2 zugeführt und dieses auf etwa 600 °C erhitzt wird. Zugleich wird das Ventil V1 geschlossen, das Ventil V2 geöffnet und die Filterkammer 1 mit einem Inertgas, hier Stickstoff $N_2$, beströmt. Die an die Rußpartikel auf der Oberfläche des Filterelements 2 angelagerten Kohlenwasserstoffe werden durch die erhöhte Temperatur freigesetzt und in dem Analysensystem 10 dem Flammenionisationsdetektor zur Bestimmung des Kohlenwasserstoffgehalts KW zugeführt.

Betriebsphase 3: Das Ventil V1 bleibt geschlossen, das Ventil V2 wird geschlossen, das Ventil V3 wird geöffnet und der Filterkammer 1 wird Luft zugeführt, mit der der auf dem Filterelement 2 angesammelte Ruß zu $CO_2$ verbrannt wird. Aus dem dann gemessenen $CO_2$-Gehalt läßt sich die Menge des in der Zeiteinheit mitgeführten Kohlenstoffs, also der Rußpartikel, bestimmen.
Danach wird der Schalter 13 wieder geöffnet, ein neuer Meßzyklus kann beginnen.

**Patentansprüche**

1. Einrichtung zur Aufbereitung von mit Rußpartikeln beladenen Abgasen für die Analyse, **gekennzeichnet durch:**

- eine Filterkammer (1) mit Anschlüssen zur Gaszu- und -abführung,
- ein darin angeordnetes, aus elektrisch leitendem, korrosionsfestem und temperaturbeständigem Werkstoff bestehendes, hohlzylindrisches, einseitig geschlossenes Filterelement (2) mit einer Porenweite im µm-Bereich,
- Anschlüsse (5) zur Zuführung elektrischer Energie an das als Widerstandsheizung betreibbare Filterelement (2),
- Zuführungen (11, 12) für Luft und/oder Inertgas in die Filterkammer (1),
- Absperrmittel (V1, V2, V3) in den Gaszuführungen (7, 11, 12).

2. Einrichtung nach Anspruch 1, **dadurch gekennzeichnet,**
- daß das Filterelement (2) aus Metallgewebe oder -vlies besteht mit einer Porenweite von etwa 5 µm.

3. Einrichtung nach Anspruch 1, **gekennzeichnet durch:**
- eine zylindrische Filterkammer (1) aus elektrisch isolierendem Werkstoff,
- ein koaxial darin angeordnetes Filterelement (2), welches mit einem Ende auf ein durch eine Stirnseite der Filterkammer (1) nach außen geführtes metallisches Rohr (3) gesteckt und befestigt und mit dem anderen Ende auf einen durch die andere Stirnseite der Filterkammer (1) nach außen geführten Metallstab (4) gesteckt und befestigt ist.

4. Einrichtung nach Anspruch 1 und 3, **gekennzeichnet durch:**
- Anschlüsse (5) zur Zuführung elektrischer Energie an dem Rohr (3) und dem Metallstab (4).

5. Einrichtung nach Anspruch 1 oder 4, **dadurch gekennzeichnet,**
- daß die Anschlüsse (5) mit einer in einem vorgebbaren Zeittakt ein- und ausschaltbaren elektrischen Energiequelle (6) mit einstellbarer Stromstärke verbunden sind.

EP 0 511 583 A2